# EUROPEAN PATENT APPLICATION

(11) **EP 1 433 854 A1**
(43) Date of publication of application: **30.06.2004**
(21) Application number: 02447267.2
(22) Date of filing: 23.12.2002
(51) Int. Cl.: C12N 15/82, A01H 5/00

(54) **Genetically modified plant having an accelerated flowering**

(71) Applicant: UNIVERSITE LIBRE DE BRUXELLES, B-1050 Bruxelles (BE)
(72) Inventor: Baucher-Martens, Marie, B-1050 Bruxelles (BE); El Jaziri, Mondher, B-1150 Bruxelles (BE)
(74) Representative: Van Malderen, Joelle

(57) **Abrégé**

La présente invention se rapporte à un procédé d'induction de la floraison dans une plante ou dans une cellule de plante, qui comprend les étapes suivantes:
- préparation d'une construction nucléotidique comprenant une séquence nucléotidique pour une protéine de la famille des annexines, ladite séquence étant sous le contrôle d'une ou plusieurs séquences de régulation génétique actives dans une plante,
- transformation de la cellule de plante avec ladite construction nucléotidique et
- éventuellement, régénération de la plante transgénique à partir de la cellule de plante transformée.

Un autre aspect de la présente invention est relatif à ladite plante transgénique ou la cellule de plante transgénique.

## Description

### Objet de l'invention

La présente invention se rapporte à une plante (ou une cellule) apte à former une plante génétiquement modifiée et présentant suite à une surexpression de l'annexine un phénotype particulier caractérisé par une floraison accélérée, une réduction du développement des racines, des tiges et des feuilles, ainsi qu'au procédé d'obtention d'une telle cellule.

### Arrière-plan technologique et état de la technique à la base de l'invention

### Diversité des annexines végétales

Les protéines de type annexine se caractérisent par leurs propriétés de se fixer aux phospholipides d'une manière calcium-dépendante (Clark et al., 2001). Elles sont présentes chez tous les eucaryotes à l'exception des champignons y compris les levures. Les annexines se retrouvent en abondance chez les végétaux.

Plusieurs annexines ont été identifiées chez un grand nombre de végétaux (Delmer et Potikha, 1997). Chez *Arabidopsis thaliana,* huit annexines différentes ont été identifiées jusqu'à présent (Clark et al., 2001).

### Structure des annexines végétales

La séquence des annexines est caractérisée par une région N-terminale variable en longueur et en composition en acides aminés, à l'encontre de la région c-terminale qui est plutôt conservée (de Carvalho Niebel et al., 1998). La région C-terminale est composée de quatre domaines de 70 à 75 acides aminés. Ces domaines possèdent chacun une région de 17 acides aminés, portant le nom *d'endonexin fold*. Cette région possède un site de liaison au calcium, correspondant au motif GXGTD. Ce motif se retrouve dans les quatre domaines à des degrés de conservation plus ou moins variables. La diversification observée dans la région N-terminale, ainsi que dans les régions situées entre les domaines, intervient probablement dans la multiplicité des fonctions que les annexines accomplissent. La partie N-terminale peut comprendre des sites potentiels de phosphorylation, de liaison à l'actine-F, à l'hème des peroxydases ou à la guanosine triphosphate (GTP).

La structure tridimensionnelle a été établie pour l'annexine 24 (X93308) de *Capsicum annuum.* Elle s'organise en quatre domaines, chacun de ces domaines comprend cinq hélices α qui forment ensemble une superhélice (Hofmann et al., 2000).

### Fonctions biologiques possibles des annexines végétales

Peu de choses sont décrites sur la fonction des annexines végétales. Différents rôles ont été proposés sur base de ce qui est connu chez les animaux (Delmer et Potikha, 1997).

La liaison des annexines aux phospholipides indique un rôle possible dans le processus de sécrétion. Ainsi, il a été démontré que les annexines p33 (X98244) et p35 (X98245) du maïs induisent l'agrégation des liposomes et des vésicules de sécrétion (Blackbourn et Battey, 1993). De plus, des annexines ont été localisées dans des cellules sécrétrices situées dans la coiffe racinaire, dans le tissu vasculaire ou dans l'épiderme des plumules du petit pois (Clark et al., 1992).

Il a été démontré que l'annexine p34 (U73747) du coton est capable d'interagir avec la callose synthase (Andrawis et al., 1993). La callose synthase est une protéine membranaire, responsable de la synthèse de callose, un glucan de type -1,3 à partir d'UDP-glucose. La production de callose s'effectue en différentes étapes qui ne sont probablement pas accomplies par une seule protéine (Verma et Hong, 2001). D'autres polypeptides tels que les annexines, pourraient être impliqués dans ce processus et intervenir dans la régulation de l'activité catalytique de l'enzyme.

Un ensemble de protéines, dans lequel se retrouve l'annexine p34, interagit avec la membrane en présence de calcium et réduit ainsi l'activité de l'enzyme (Andrawis et al., 1993). Des expériences *in vivo* montrent par contre une activation de la callose synthase, par l'augmentation du niveau de calcium (Verma et Hong, 2001). La régulation de l'enzyme *in vivo* est probablement beaucoup plus complexe et ne repose pas uniquement sur le niveau de Ca²⁺ intracellulaire. Il est possible, que l'interaction entre l'annexine et la callose synthase détermine la localisation de l'enzyme dans la cellule, ou permet son ancrage au cytosquelette. Dans ce cas, l'annexine n'influencerait pas l'activité de la callose synthase (Verma et Hong, 2001).

La phosphorylation des annexines des mammifères a été observée sur des résidus tyrosine et serine par la protéine kinase C. Chez les végétaux en général, une phosphorylation se fait surtout au niveau des résidus thréonine et sérine (Delmer et Potikha, 1997).

Chez les annexines AnnAt1 (AF083913) de *A*. *thaliana,* AnnGh2 (U73747) du coton et p33 du maïs, un motif T-L-K peut être distingué du côté N-terminal (Delmer et Potikha, 1997). Ce motif est similaire au motif reconnu par les protéines kinases C chez les annexines des mammifères et constitue donc un site potentiel de phosphorylation (Raynal et Pollard, 1994). L'exemple unique de phosphorylation des annexines végétales est présenté par l'annexine AnnGh2.

De plus, de nombreuses annexines de mammifères ont la capacité de former un canal calcique (Delmer et Potikha, 1997). Cette fonction a été suggérée pour l'annexine p39 du blé (Breton et al., 2000). L'annexine 24 du poivron montre aussi une telle activité, in vitro (Hofmann et al., 2000).

Les annexines p34 (AF079232) et p35 (AF079231) de la tomate sont capables, *in vitro,* d'interagir avec l'actine-F, à condition qu'un certain niveau de calcium soit atteint (Calvert et al., 1996). Il a également été démontré que les annexines Ntp32.1 (Y14972) et Ntp32.2 (Y14973), identifiées chez le tabac, interagissent avec l'actine-F (Proust et al. 1998).

Une activité de nucléotide phosphodiestérase, qui permet d'hydrolyser des nucléosides di- et triphosphates, a été mise en évidence chez les annexines p34 et p35 de la tomate (Calvert et al., 1996).

Chez *A*. *thaliana,* l'annexine ANXarb (U28415), identifiée lors de l'étude de mutants peroxydase *d'Escherichia coli,* exprime une activité peroxydase. La séquence N-terminale du gène *ANXarb* montre une homologie avec une région conservée, présente chez d'autres peroxydases végétales (Gidrol et al., 1996).

### Expression spatio-temporelle des annexines végétales

Sept gènes codant pour les annexines AnnAt1-7, d'une masse moléculaire approximative de 36 kDa, et se caractérisant par des points isoélectriques variant entre 5.0 et 9.5, ont été identifiés chez *A*. *thaliana* (Clark et al., 2001). Une étude par RT-PCR a démontré que l'expression de ces gènes n'est pas spécifique à un organe particulier de la plante, sauf en ce qui concerne *AnnAt6* (AY014798) dont l'expression semble être limitée aux fleurs (Clark et al., 2001). L'expression *d'AnnAt1* et *d'AnnAt2* (AF083914) a été comparée dans différents organes végétaux de plantes âgées de 2,5 semaines par RT-PCR. *AnnAt1* est fortement exprimé dans les tiges, alors que le transcrit *d'AnnAt2* se retrouve en majorité dans les racines. Le transcrit *d'AnnAt2* n'a pas été détecté dans les feuilles. Le transcrit *d'AnnAt1* par contre est présent d'une manière abondante dans les jeunes feuilles (Clark et al., 2001).

Chez *N. tabacum,* l'expression des gènes *Ntan.1* (Y17502) et *Ntan.2* (Y17503) qui codent pour les annexines Ntp32.1 et Ntp32.2, a été étudiée dans les différents organes végétaux (Proust et al., 1999). Ces annexines n'ont pas été détectées dans les feuilles âgées et sont présentes à un niveau faible dans les jeunes feuilles et les racines. Par contre, elles sont accumulées dans les tissus composés de cellules mitotiquement actives, tels que les bourgeons floraux et les jeunes tiges (Proust et al., 1999).

L'annexine codée par le gène *AnnMs* (X74947), identifiée chez *Medicago sativa* est présente dans tous les organes de la plante à des niveaux d'expression variables (Pirck et al., 1994). Les transcrits *d'AnnMs2* (Y11348), un gène codant pour une annexine induite par des facteurs de stress, tels qu'un stress salin, hydrique ou osmotique, chez *M. sativa,* ont été détectés en abondance dans les fleurs, les bourgeons floraux et les racines par *Northern* blot. Il a également été démontré que les transcrits étaient moins abondants dans les tiges et les feuilles (Kovacs et al., 1998).

Une étude de l'expression génique des annexines a été effectuée dans la tomate, la pomme de terre et l'orge à l'aide d'un fragment PCR développé sur base de la séquence de l'annexine p34 identifiée chez la tomate. Chez ces trois plantes, les niveaux d'expression les plus élevés ont été observés dans les racines. Les tiges et les jeunes feuilles sont caractérisées par des niveaux d'expression inférieurs. Aucune expression n'a été détectée dans les feuilles plus âgées (Smallwood et al., 1992).

L'expression de certaines annexines est associée à des stades de développement particuliers. C'est notamment le cas de deux annexines, RJ4 (AF188832) et Ca32 (X93308), identifiées chez la fraise et le poivron respectivement. Par *differential display,* il a été démontré que *RJ4* n'est que faiblement exprimé dans les fruits verts. Son expression augmente lors de la maturation des fruits pour atteindre un niveau maximal dans les fruits rouges matures. Elle n'a pas été détectée dans les autres organes de la plante (Wilkinson et al., 1995). Chez le poivron, l'annexine 24 a également été identifiée au cours du développement du fruit. Le niveau de transcrits dans les fruits rouges est 3.5 fois plus élevé que dans les fruits verts (Proust et al., 1996).

L'annexine p35 du petit pois a été localisée dans des tissus contenant des cellules sécrétrices tels que l'épiderme, le tissu vasculaire et la coiffe racinaire (Clark et al., 1992).

Par hybridation *in situ*, l'expression d*'AnnAt1* et *AnnAt2* de *A*. *thaliana* a été étudiée dans les différents tissus au cours du développement de la plantule (Clark et al., 2001). Dans les premiers stades du développement, les annexines AnnAt1 et AnnAt2 ont été détectées dans les cellules épidermiques de la racine. L'expression d'*AnnAt1* est limitée aux cellules épidermiques de la zone d'élongation de la racine, alors que le transcrit *AnnAt2* a été observé dans les cellules épidermiques de la racine, des cotylédons et au sommet de l'hypocotyle. Dans les stades de développement ultérieurs, le transcrit *AnnAt1* est situé dans l'ensemble des cellules de la racine. Il est aussi présent dans les poils racinaires. Le transcrit d*'AnnAt2* est situé dans les cellules de l'endoderme, à proximité de la jonction entre la racine et l'hypocotyle, dans les cellules de l'épiderme, à proximité de l'apex et dans les racines latérales. Dans les pousses plus matures, le transcrit *d'AnnAt1* a été retrouvé en premier lieu dans les tissus vasculaires, dans les tissus à la base des feuilles et des tiges, ainsi que dans les trichomes. L'expression *d'AnnAt2* se limite aux cellules épidermiques qui se situent entre les méristèmes et les primordia (Clark et al., 2001).

Certaines annexines végétales sont induites par des facteurs de stress biotiques, tels que la présence d'un pathogène (de Carvalho Niebel et al., 1998) ou abiotiques tels qu'un traitement salin (Kovacs et al., 1998).

Par *differential display,* le gène codant pour l'annexine MtAnn1 (Y15036) a été mis en évidence chez M. *truncatula* suite à une infection par *Rhizobium meliloti* (de Carvalho Niebel et al., 1998). L'infection d'une plante par ce pathogène conduit à la formation d'une structure nodulaire au niveau des racines. *MtAnn1* est un gène 'Précoce', dont l'expression est induite relativement tôt dans le processus de nodulation. Cette annexine a seulement été détectée dans le nodule et dans les racines. Toutefois, son expression ne semble pas être liée au processus d'infection. Elle intervient plutôt dans les phases préliminaires à l'infection, vu que le mutant Nod⁻ de R. *meliloti* est toujours capable d'induire l'expression du gène *MtAnn1.* L'induction de l'expression de *MtAnn1* semble néanmoins être spécifique à *R. meliloti,* puisque ceci n'est pas observé dans d'autres conditions de stress, d'origine biotique telles que l'infection par *Pseudomonas syringae,* ou abiotique telles qu'une blessure (de Carvalho Niebel et al., 1998).

Le gène *NtanNH12,* codant pour une annexine, a été isolé dans des cellules de tabac BY-2 infectées par R. *fascians* (El Hassan, 2002). Le transcrit de cette annexine, mis en évidence par RT-PCR, est également présent dans des cellules de tabac en phase stationnaire de la culture, mais à un niveau inférieur. Cette annexine semble donc être induite par des conditions de stress abiotiques et/ou biotiques.

Chez *M. sativa,* le gène *AnnMs2* codant pour une annexine a été isolé lors de l'étude de gènes exprimés dans des conditions de stress (Kovacs et al., 1998). Une augmentation du niveau de transcrits a été observée suite à un stress osmotique, hydrique ou salin. Ces facteurs de stress agissent par l'intermédiaire de l'acide abscissique qui, lui aussi, provoque une augmentation de l'expression *d'AnnMs2* (Kovacs et al., 1998).

Finalement, dans des plantes d'*A*. *thaliana* âgées de quatre semaines, une augmentation du niveau d'ARNm de l'annexine ANXarb a été observée en réponse à un traitement au peroxyde (Gidrol et al., 1996).

### Localisation subcellulaire des annexines végétales

Par immunolocalisation, il a été démontré que les annexines végétales sont localisées dans différents compartiments cellulaires : au niveau de la membrane intracellulaire (Breton et al., 2000), du cytoplasme (Thonat et al., 1997), du noyau (Clark et al. 1998) ou de la vacuole (Seals et al., 1997). Dans le cytoplasme, elles sont souvent associées à l'appareil de Golgi (Clark et al., 1992).

L'annexine VCaB42 a été identifiée chez le céleri par son association calcium-dépendante avec la membrane de la vacuole. Il est supposé que cette protéine détecte le niveau de calcium dans le cytosol et qu'elle transmet cette information à la vacuole (Seals et al., 1994).

Par immunolocalisation, l'annexine p35 a été détectée à la périphérie des cellules, ainsi que dans le noyau des cellules épidermiques des plumules du petit pois (Clark et al., 1998). La distribution de cette annexine dans la cellule est asymétrique et semble prédominante selon la direction de la gravité. Une concentration plus importante de l'annexine p35 à la périphérie de la cellule a été observée après gravistimulation (Clark et al., 2000). Dans le cas de *Bryonia dioica,* une redistribution des annexines du cytoplasme vers la surface cellulaire a été démontrée suite à des stimuli mécaniques (Thonat et al., 1997).

Deux groupes d'annexines ont été identifiés chez le blé (Breton et al., 2000). Le premier groupe comprend les annexines p36 et p38 qui se localisent dans le cytosol et requièrent du calcium afin d'établir une liaison avec des phospholipides membranaires. Le deuxième groupe comprend les annexines p39 et p22.5. Il est supposé que l'état intrinsèque des annexines p39 et p22.5 au niveau de la membrane est induit par des interactions spécifiques protéine-protéine ou par phosphorylation. De plus, il a été démontré que l'annexine p39 est localisée au niveau de la membrane plasmique (Breton et al., 2000).

L'expression des gènes codant pour les annexines AnnMs2, Ntp32.1 et Ntp32.2 a été étudiée au cours du cycle cellulaire. L'annexine AnnMs2, identifiée dans *M*. *sativa,* montre une distribution cellulaire variable au cours des différentes phases du cycle cellulaire. Dans les cellules qui se trouvent en interphase, elle est localisée dans le cytoplasme, au niveau du cortex nucléolaire et dans le perinucleus. Dans les cellules en phase de mitose, elle se situe plutôt au niveau des chromosomes. Vers la fin de la mitose et après la formation de la membrane nucléaire, elle est à nouveau détectée dans la zone perinucléaire et cytoplasmique (Kovacs et al., 1998).

Les annexines Ntp32.1 et Ntp32.2, isolées à partir des cellules de tabac BY-2, sont exprimées durant la phase de croissance exponentielle de la suspension cellulaire et ne sont pas détectées durant la phase stationnaire. L'expression des annexines Ntp32.1 et Ntp32.2 est également modulée au cours du cycle cellulaire. Elles sont exprimées lors des phases de transition G2/M et G1/S ainsi que durant la phase de mitose. Ces annexines Ntp32.1 et Ntp32.2, identifiées dans les cellules de tabac BY-2, se localisent en majorité au niveau des jonctions intercellulaires et forment ainsi une structure annulaire en dessous de la membrane plasmique (Proust et al., 1999).

### Buts de l'invention

La présente invention vise à obtenir une plante (y compris un arbre) ou une cellule (apte à reconstituer ladite plante) génétiquement modifiées et présentant des caractéristiques phénotypiques améliorées, en particulier une floraison accélérée (en particulier in *vitro*) et éventuellement d'autres caractéristiques spécifiques telles qu'une réduction du développement racinaire, une réduction de la taille et de la croissance des tiges et des feuilles.

Un autre aspect de la présente invention vise à obtenir un procédé simple et rapide de modification génétique desdites plantes (et desdites cellules) présentant une floraison accélérée, en particulier *in vitro* quelle que soit la saison de développements des fleurs d'une plante, en particulier celles des arbres ou des vignes.

### Eléments caractéristiques de l'invention

Le premier aspect de l'invention concerne un procédé d'induction de la floraison dans une plante ou dans une cellule de plante apte à reformer ladite plante qui comprend les étapes suivantes:
- préparation d'une construction nucléotidique comprenant une séquence nucléotidique codant pour une protéine ou une portion active d'une protéine de la famille des annexines, l'expression de ladite séquence étant sous le contrôle d'une ou plusieurs séquences de régulation génétique active dans une plante.
- transformation d'une cellule de ladite plante avec ladite construction nucléotique et éventuellement régénération de la plante transgénique à partir de la cellule de plante transformée.

Un autre aspect de la présente invention concerne ladite constuction nucléotidique ainsi que ladite cellule ou plante génétiquement modifiée présentant une floraison accélérée et incorporant ladite construction nucléotidique. Les portions actives des protéines annexines encodées par la séquence génétique de ladite construction nucléotidique sont notamment la région *endonexin fold* de plus ou moins 17 acides aminés, le site de liaison au calcium (GXGTD), les acides aminés impliqués dans la liaison à l'hème des peroxydases, le motif IRI de liaison à l'actine-F et le résidu tryptophane impliqué dans une probable liaison aux phospholipides (Clark et al., 2001).

Selon l'invention, ladite séquence nucléotidique code de préférence pour l'annexine *NtanNH12* (SEQ. ID N° 1) ou une portion active de celle-ci ainsi que ses homologues présentant de préférence une homologie supérieure à 70 %, 80 %, 90 %, 95 % ou 98 % avec la séquence SEQ. ID N° 1, les portions actives de cette protéine étant celles décrites ci-dessus.

Avantageusement, la plante est choisie parmi le groupe constitué par le gymnosperme et les angiospermes, en particulier les arbres (y compris les vignes).

Selon l'invention, la séquence régulatrice est une séquence promotrice et/ou terminatrice active dans une plante (de préférence une séquence régulatrice inducible), telle qu'un promoteur consécutif ou un promoteur étranger, par exemple, le promoteur 35S Cauliflower Mosaic Virus ou le promoteur Polyubiquition Arabidopsis thaliana. De préférence, ces séquences régulatrices sont choisies pour provoquer une surexpression des gènes dans ladite cellule et la plante.

Le dernier aspect de la présente invention est relatif à un tissu ou organe de plante transgénique choisi parmi le groupe constitué par les fruits, les bourgeons , les racines, les fleurs, les semences ou toute structure reproductible obtenue à partir de ladite plante transgénique ou ladite cellule transgénique de l'invention.

La présente invention sera décrite ci-dessous sous une forme d'exécution préférée en référence aux figures annexées; cette forme d'exécution préférée étant présentée à titre d'illustration non limitative de l'invention.

### Description détaillée d'une forme d'exécution préférée de l'invention.

### Surexpression des gènes NtanNH12 dans N. tabacum

Différentes étapes ont été effectuées afin de réaliser la surexpression du gène *NtanNH12* dans *Nicotiana tabacum.* La technologie de clonage Gateway™, composée des réactions de recombinaison site-spécifique BP et LR a conduit à l'intégration de la séquence codante de *NtanNH12* dans un vecteur de destination, pK7WG2 (Karimi et al., 2002). Le vecteur de destination est exprimé dans des cellules compétentes DH5 d'*E. coli* qui sont alors qualifiées de clones d'expression. Une transformation d'une souche (PGV2260) d'*Agrobacterium tumefaciens* a été effectuée avec l'ADN plasmidique purifié à partir d'un clone d'expression. La régénération de lignées de tabac a été obtenue, suite à l'infection d'explants de feuilles de tabac par des colonies transformées de la souche PGV2260. L'intégration du gène marqueur de sélection ainsi que du transgène, a été vérifiée par PCR et par RT-PCR.

Des amorces permettant d'introduire les sites *attB* aux extrémités de la séquence codante de *NtanNH12,* ont été développées sur base de la séquence nucléotidique de *NtanNH12.* Les codons start et stop de la séquence codante de *NtanNH12* doivent obligatoirement être présents dans la construction réalisée afin d'indiquer le début et la fin de la traduction, mais la phase de lecture ne doit pas être maintenue.

La réaction BP a été effectuée à l'aide du fragment PCR *NtanNH12-attB* purifié à partir d'un gel d'agarose. L'intégration du cDNA *NtanNH12* dans le vecteur donneur pDONR™201 et son expression dans *E. coli,* ont été confirmées par PCR et par séquençage de l'insert.

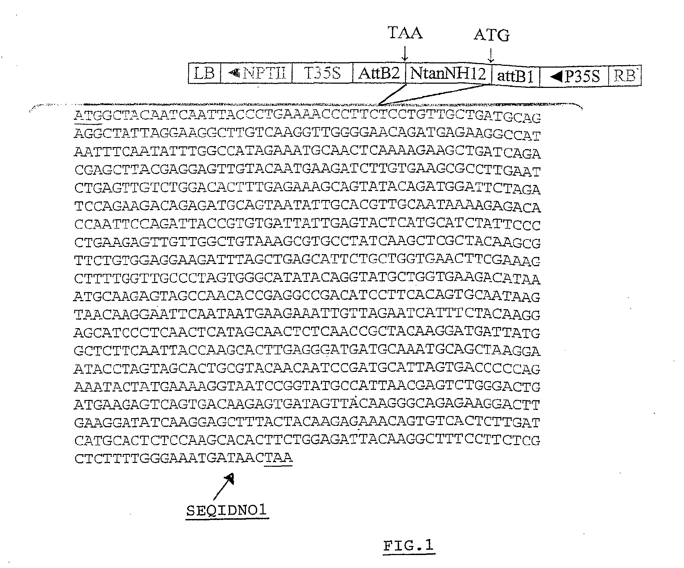

La réaction LR, aboutissant à l'intégration du cDNA dans le vecteur de destination pK7WG2 (Fig. 1), a été réalisée à l'aide de l'ADN purifié à partir d'un clone d'entrée sélectionné AnnBP1.

Des PCR réalisées sur cinq clones d'expression, obtenus par la réaction LR, ont confirmé la présence du gène *NtanNH12.* Un clone d'expression (AnnLR1) a été retenu pour la suite de l'expérience.

### Transformation de la souche PGV2260 d'Agrobacterium tumefaciens

La transformation de la souche PGV2260 par l'ADN plasmidique purifié à partir du clone AnnLR1, a été effectuée par la méthode à l'azote liquide. Les colonies de PGV2260 qui ont incorporé le vecteur de destination pK7WG2 ont été sélectionnées sur un milieu contenant les antibiotiques ampicilline, rifampicine, spectinomycine et streptomycine. Une colonie (p35-Ann) a été retenue pour la suite de l'expérience.

### Transformation et régénération des lignées de tabac in vitro

Des plantes de tabac transgénique ont été régénérées in vitro après infection d'explants de feuilles de tabac par une culture de la colonie p35-Ann (tel que décrit par Leplé et al., 1992). Après un traitement hormonal (TDZ et NAA) et une culture de six semaines sur milieu additionné de kanamycine, les bourgeons obtenus ont été découpés de l'explant initial et transférés sur milieu frais dépourvu de kanamycine. Les plantes régénérées ont ensuite été multipliées par micro-bouturage *in vitro.*

### Confirmation de la transformation génétique des plantes sélectionnées

Parmi les plantes de tabac régénérées *in vitro,* 9 lignées présentant une quantité de matériel végétal suffisante, ont été sélectionnées. Le gène *nptII* a été mis en évidence par PCR dans ces plantes. Au neuf plantes déjà sélectionnées, se sont ajoutées par la suite trois plantes supplémentaires. Une étude des transcrits de *nptII* et du transgène *NtanNH12* a été effectuée sur 12 plantes de tabac régénérées *in vitro* ainsi que sur une plante de tabac contrôle.

### Vérification de la transformation des lignées de tabac sur base de l'ADN génomique

L'intégration du gène *nptII* dans le génome a été mise en évidence pour chaque plante de tabac analysée, à l'exception du contrôle. Une variation dans l'intensité des bandes PCR pour les 9 lignées étudiées pourrait être attribuée à l'intégration d'un nombre de copies de cDNA de *nptII* variable entre les lignées.

### Etude de l'expression de NtanNH12 et de nptII dans les lignées de tabac transgénique

L'étude de l'expression de *nptII* et de *NtanNH12* a été réalisée par la mise en évidence des transcrits *nptII* par RT-PCR.

L'ARN total a été extrait de douze lignées de tabac et la qualité de l'ARN a été vérifiée sur gel. La bande supérieure, correspondant à l'ARN ribosomial 25S, montre une intensité au moins égale à celle de la bande inférieure correspondant à l'ARN ribosomial 18S.

L'expression du transgène *NtanNH12* a été mise en évidence par RT-PCR en utilisant des amorces correspondant aux sites attB complets.

### Analyse du phénotype des lignées de tabac transgénique

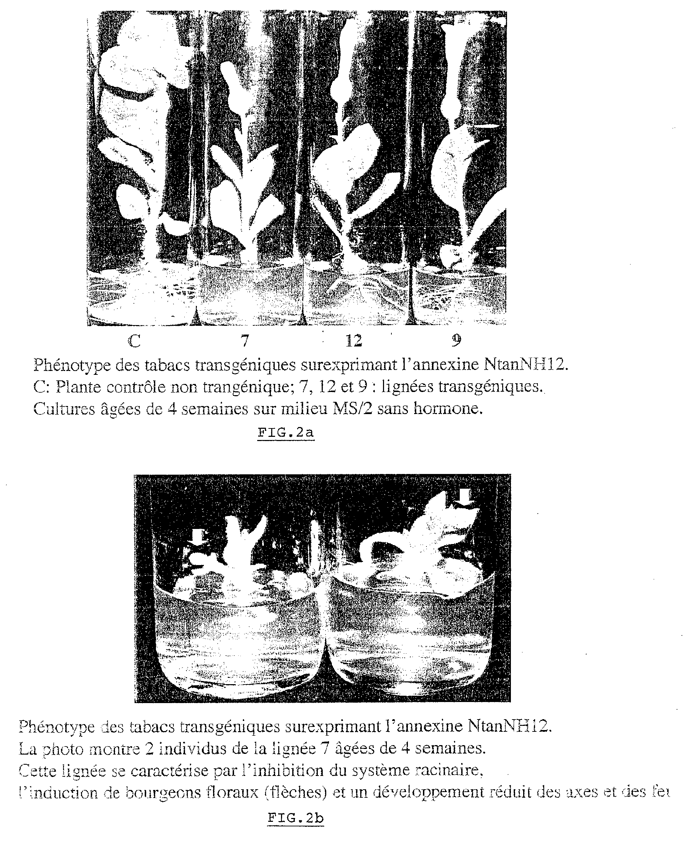

Le phénotype des lignées de tabac transgénique a été observé au niveau de jeunes plantules régénérées et enracinées *in vitro* (Fig. 2).

Le phénotype des plantes montre une floraison spontanée et une réduction des tiges, des feuilles et/ou des racines.

### Localisation subcellulaire de NtanNH12

Dans le but de mettre en évidence la localisation subcellulaire de NtanNH12 dans des cellules de tabac BY-2, le gène codant pour la *Green Fluorescent* Protein (GFP) a été utilisé comme gène rapporteur. La technologie de clonage Gateway™ a conduit à l'intégration du cDNA *NtanNH12* dans les trois vecteurs de destination suivants : pK7FWG2, pK7WGF2 et pK7FWGF2. Les vecteurs de destination ont été par la suite exprimés dans les cellules compétentes DH5 d'*E. coli* qui sont alors qualifiées de clones d'expression. La souche virG d'*A*. *tumefaciens* a été ensuite transformée avec l'ADN plasmidique purifié à partir de trois clones d'expression, exprimant chacun un vecteur de destination différent.

Des cals BY-2 transgéniques ont finalement été obtenus suite à l'infection de cellules de tabac BY-2 par des colonies transformées de la souche virG, selon la technique décrite par Geelen D., 2000.

### Construction génétique de la fusion entre le cDNA NtanNH12 et le cDNA GFP

Des amorces permettant d'introduire les sites *attB* aux extrémités de la séquence codante de *NtanNH12,* ont été mises au point sur base de la séquence nucléotidique de *NtanNH12.* Le maintient de la phase de lecture correcte est essentiel dans ce type de construction. Selon l'extrémité du cDNA *NtanNH12* à laquelle la séquence codante de GFP est fusionnée, le codon stop est présent ou absent dans le cDNA *NtanNH12* intégré dans les vecteurs de destination. Dans le cas de la fusion du cDNA GFP à l'extrémité N de la séquence codante de *NtanNH12,* le codon stop doit être présent afin d'indiquer la fin de la traduction. Le codon start du cDNA *NtanNH12* ne doit pas être éliminé de la séquence ; celui-ci permet alors également la traduction de *NtanNH12* non fusionnée, à côté de la traduction de la protéine fusionnée. Le cDNA *NtanNH12* obtenu pour la fusion au cDNA *GFP* du côté N-ou N- et C-terminal ne contient pas de codon stop. La fin de la traduction est indiquée par un codon "stop" présent dans le cDNA *GFP.*

Deux réactions BP ont été effectuées à l'aide des fragments PCR *NtanNH12-attB* purifié à partir d'un gel d'agarose. La présence du cDNA *NtanNH12* dans les vecteurs donneurs pDONR™201 est confirmée par PCR et par séquençage de l'insert.

La réaction LR, aboutissant à l'intégration du cDNA *NtanNH12* dans les vecteurs de destination pK7FWG2, pK7WGF2 et pK7FWGF2, a été réalisée à l'aide de l'ADN plasmidique purifié à partir de deux clones d'entrée AnnBP2 et AnnBP3.

L'intégration du cDNA *NtanNH12* dans les vecteurs de destination est confirmée par PCR. Le premier clone d'expression exprimant le vecteur pK7WGF2 (AnnLR2) a été choisi. Parmi les deux clones d'expression exprimant le vecteur pK7FWG2, le deuxième (AnnLR3) a été sélectionné. Le deuxième clone d'expression (AnnLR4) qui a incorporé le vecteur pK7FWGF2, a été retenu pour la suite de l'expérience.

### Transformation de la souche virG d'Agrobacterium tumefaciens

La transformation de la souche virG d'*A*. *tumefaciens* par l'ADN plasmidique purifié à partir des clones AnnLR2, AnnLR3 et AnnLR4 a été effectuée par électroporation. Les colonies transformées, GFP-Ann, Ann-GFP et GFP-Ann-GFP sont sélectionnées sur un milieu sélectif (gentamycine, spectinomycine et streptomycine).

### Transformation des cellules de tabac BY-2

Une co-culture de cellules de tabac BY-2 et de colonies GFP-Ann, Ann-GFP et GFP-Ann-GFP, a conduit à l'obtention de cellules de tabac transgénique. Les cals développés à partir de ces lignées cellulaires ont été repiqués hebdomadairement sur un milieu frais.

### Analyse de la localisation subcellulaire de l'annexine NtanNH12 par fusion avec la GFP

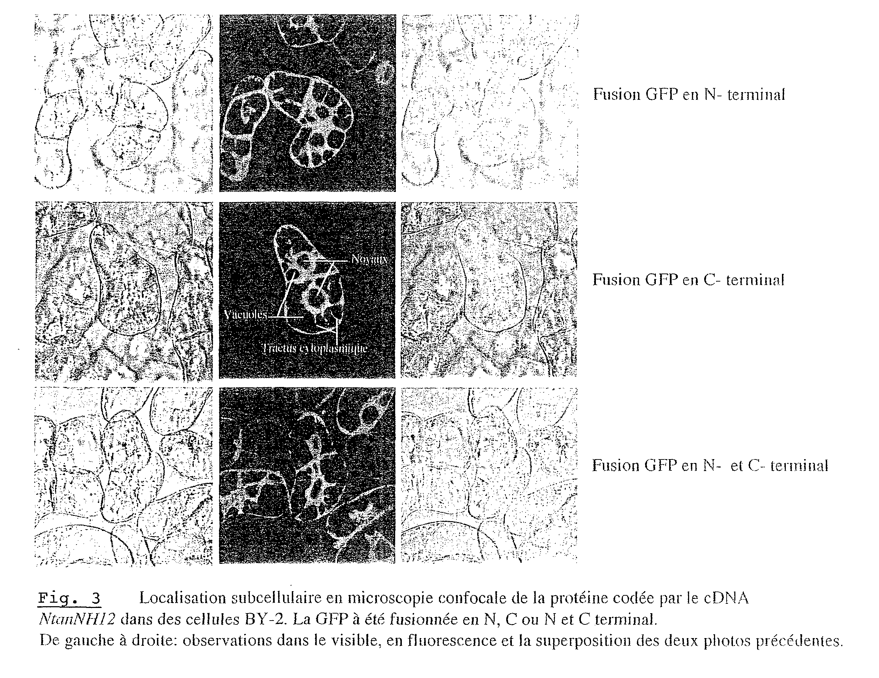

Une fluorescence mesurée à l'aide d'un microscope confocale (Axcourt 100M, Zeiss) au niveau cytoplasmique et nucléaire est observée lorsque la protéine GFP (excitée à 488 nm par un laser argon et émission enregistrée entre 510 et 530 nm) non fusionnée se retrouve dans une cellule de tabac BY-2 (Zuo J. et al., 2000). L'analyse des cals transgéniques de BY-2 a révélé une localisation cytoplasmique de la protéine de fusion NtanNH12-GFP. La position de la GFP par rapport à l'annexine (N-, C- ou N- et C-terminal) ne semble pas modifier la localisation cytoplasmique de la protéine de fusion (Fig. 3).

### Surexpression du gène NtanNH12

L'analyse par PCR de l'ADN génomique a confirmé la présence du gène marqueur de sélection *nptII* dans le génome des lignées régénérées après transformation par *A. tumefaciens.* L'analyse par RT-PCR a révélé des niveaux d'expression variable de *NtanNH12* pour les différentes lignées transgéniques sélectionnées au niveau plantule. Cette analyse a révélé également que le transcrit de *NtanNH12* n'a pas été détecté dans la plante contrôle. Toutefois, des transcrits de NtanNH12 ont été mis en évidence dans les tissus de la galle feuillée.

Un examen de la littérature révèle que toutes les annexines de plantes, connues à ce jour s'expriment dans les organes végétaux soit d'une manière plus ou moins constitutive (Clark et al., 2001) soit d'une manière tissu spécifique (Wilkinson et al., 1995). L'annexine NtanNH12 semble se distinguer de l'ensemble des annexines décrites dans la littérature dans la mesure où son expression n'a pas été mise en évidence dans les différents organes de la plante (graines, fleurs, racines, tiges, feuilles).

NtanNH12 et MtAnn1 (Mtannexin) sont les deux seuls exemples d'annexines dont l'expression est induite suite à une interaction avec un micro-organisme, *Rhodococcus fascians-*BY-2 et *Rhizobium meliloti-Medicago* truncatula, respectivement (de Carvalho Niebel et al., 1998). L'expression de *NtanNH12* est également induite dans des conditions de stress abiotiques associées à un appauvrissement du milieu de culture et à une augmentation de la biomasse cellulaire en phase stationnaire de cultures de cellules BY-2.

D'autres annexines ont été mises en évidence dans des conditions de stress. C'est le cas de Msnann (Y11348), une annexine identifiée chez *M. sativa* et dont l'expression est modulée par des conditions de stress osmotique, hydrique ou salin ou par l'ABA (Kovacs et al., 1998). Chez *A*. *thaliana,* une augmentation du niveau d'expression de l'annexine ANXarb (U28415) a été observée suite à un traitement au peroxyde (Gidrol et al., 1996). Toutefois, aucune étude n'a été effectuée sur l'impact de la surexpression d'une telle annexine sur le phénotype d'une plante.

### Localisation subcellulaire de NtanNH12

L'étude de la localisation subcellulaire de NtanNH12 a été réalisée sur des lignées de BY-2 surexprimant une séquence codant pour l'annexine NtanNH12 fusionnée à la *Green Fluorescent Protein* (GFP).

La protéine GFP est originaire de la méduse Aequorea victoria. Cette protéine présente la particularité d'émettre dans le vert après absorption dans le bleu (Chalfie et al., 1994). La fluorescence qui caractérise cette protéine est stable et se maintient dans des conditions de protéolyse ou en cas de fixation chimique. La GFP s'avère donc très utile dans des études d'expression génique, de localisation subcellulaire ou de la dynamique des organelles (Hawes et al., 2001 ; Persson et al., 2002).

Les 3 fusions annexine-GFP testées (N-, C-ou N- et C-terminal) ont révélé une localisation cytoplasmique de NtanNH12 dans les cellules BY-2.

La localisation subcellulaire des annexines, aussi bien végétales qu'animales, a été le sujet d'un grand nombre d'études. Les annexines végétales ont été localisées par immunofluorescence au niveau extra- (Clark et al., 1992) et intracellulaire (Kovacs et al., 1998 ; Clark et al., 2000). Au niveau subcellulaire, elles ont été localisées dans le cytoplasme (Thonat et al., 1997), au niveau des membranes (Breton et al., 2000) dans le noyau (Clark et al., 1998) ou dans la vacuole (Seals et al., 1994).

La localisation cytoplasmique de l'annexine NtanNH12 n'est pas un cas unique. Les annexines p34 et p36 du blé ont également été retrouvées dans le compartiment cytoplasmique. Celles-ci ont été purifiées à partir de fractions protéiques, par précipitation en ajoutant du calcium et des phospholipides (Breton et al., 2000). Les annexines p33 et p35 de *B*. *dioica* ont été localisées dans le cytoplasme des cellules de parenchyme des entre-noeuds (Thonat et al., 1997). L'annexine VCaB42 a été identifiée en association avec la vacuole. Il a été suggéré qu'elle se trouve dans le cytoplasme et qu'elle s'associe au tonoplaste en fonction de la concentration intracellulaire de calcium.

Certaines conditions doivent être remplies afin de pouvoir détecter les annexines en association avec des phospholipides membranaires. Dans le cas des annexines p34 et p36 du blé et de l'annexine VCaB42 du céléri, il faut que le niveau de calcium intracellulaire atteigne un seuil critique pour que l'association avec des phospolipides membranaires s'établisse (Breton et al., 2000 ; Seals et al., 1994 ; Seals et al.,1997). L'association de l'annexine VCaB42 au tonoplaste pourrait indiquer qu'elle remplit une fonction dans les vacuoles matures (Seals et al., 1994 ; Seals et al., 1997). Chez *B*. *dioica*, une redistribution des annexines p33 et p35 du cytoplasme vers la membrane a été remarquée en réponse à des stimuli mécaniques. La redistribution des annexines p33 et p35 est liée soit aux changements de la fluidité de la membrane plasmique soit à l'augmentation de la concentration de calcium (Thonat et al., 1997).

Les annexines p35 du petit pois ont été observées dans le cytoplasme ainsi qu'à la périphérie des cellules (Clark et al., 2000).

Dans certains cas, la distribution des annexines n'est pas limitée au compartiment cytoplasmique, mais est également observée au niveau des structures nucléaires de la cellule. C'est notamment le cas de Msnann identifiée chez *M. sativa.* Elle pourrait être impliquée dans le maintient de la structure nucléolaire ou dans la synthèse d'ARN ribosomial et l'assemblage des ribosomes (Kovacs et al., 1998).

La localisation de l'annexine NtanNH12 est exclusivement limitée au cytoplasme chez des cellules de tabac BY-2. Aucune fluorescence n'a été remarquée dans le noyau, comme c'est le cas pour Msnann, induite dans *M*. *sativa* dans des conditions de stress hydrique et osmotique notamment. La protéine NtanNH12 est donc probablement impliquée dans un processus autre que celui mentionné pour l'annexine Msnann.

### BIBLIOGRAPHIE

ANDRAWIS, A., et al. (1993). *Plant J.* 3 (6), 763-772.
BLACKBOURN, H.D., and BATTEY, N.H. (1993). *Physiol. Plant* 89, 27-32.
BOUSTEAD, C.M., et al. (1989). *FEBS Lett*. 244, 456-460.
BRETON, G., et al. *Plant Cell Physiol.* 41 (2), 177-184.
CALVERT, C.M., et al. (1996). *Plant Cell* 8, 333-342.
CHALFIE, M., et al. (1994). *Science* 263, 802-804.
CLARK, G.B., et al. (1992). *Planta* 187, 1-9.
CLARK, G.B., et al. (1998). *Plant Physiol. Biochem.* 36 (9), 621-627.
CLARK, G.B., et al. (2000). *Plant Physiol. Biochem.* 38, 937-947.
CLARK, G.B., et al. (2001). *Plant Physiol.* 126, 1072-1084.
DE BLOCK (1987). *EMBO Journal* 6, 2513-2518.
de CARVALHO NIEBEL, F., et al. (1998). *Mol. Plant Microbe Interact.* 11(6), 504-513.
DELMER, D.P. and POTIKHA, T.S. (1997). *Mol. Life Sci.* 53 (6), 546-553.
GIDROL, X., et al. (1996). *Proc. Nat. Acad. Sci.USA* 93, 11268-11273.
HAWES, C., et al. (2001). *Protoplasma* 215, 77-88.
HOFMANN, A., et al. (2000). *J. Biol. Chem.* 275 (11), 8072-8082.
KARIMI, M., et al. (2002). *Trends in Plant Science* 7, 193-195.
KOURIE, J.I., and WOOD, H.B. (2000). *Progress in Biophysics & Molecular Biology* 73, 91-134.
KOVACS, I., et al. (1998). *Plant J.* 15 (2), 185-197.
LEPLE, J.C., et al. (1992). *Plant Cell Rep.* 11, 137-141.
MILLER, J.H. (1972). Experiments in Molecular genetics. New York, *Cold Spring Harbor Laboratory.*
MULLIS, K.B., et al. (1986). *Cold Spring Harbor Symp. Quant. Biol.* 51, 263-273.
MURASHIGE, T., and SKOOG, F. (1996). *Physiol. Plant.* 15, 473-497.
NAGATA, T., et al. (1992). *International review of cytology* 132, 1-30.
PERSSON, S., et al. (2002). *Plant Physiol.* 128, 341-344.
PIRCK, M., et al. (1994). *Plant Physiol.* 104, 1463-1464.
PROUST, J., et al. 996). *FEBS letters.* 383, 208-212.
PROUST, J., et al. (1999). *Biol.* 39 (2), 361-372.
SEALS, D.F., et al. (1994). *Plant Physiol.* 106, 1403-1412.
SEALS, D.F., et al. (1997). *Plant Physiol.* 115, 753-761.
SMALLWOOD, M.F., et al. (1992). *Biochem. J*. 281, 501-505.
THONAT, C., et al. (1997). *Plant Physiol.* 114, 981-988.
VERMA, D.P.S., and HONG, Z. (2001). *Plant Mol. Biol.* 47, 693-701.
WILKINSON, J.Q., et al. (1995). *Plant Mol. Biol.* 27, 1097-1108.
ZUO, J., et al. (2000). *Plant Cell* 12, 1137-1152.

## Revendications

1. Procédé d'induction de la floraison dans une plante ou dans une cellule de plante apte à reconstituer ladite plante, qui comprend les étapes suivantes :
- préparation d'une construction nucléotidique comprenant une séquence nucléotidique codant pour une protéine ou une portion active de ladite protéine de la famille des annexines, ladite séquence étant sous le contrôle d'une ou plusieurs séquences de régulation génétique actives dans une plante,
- transformation d'une cellule de ladite plante avec ladite construction nucléotidique, et
- éventuellement, régénération de la plante transgénique à partir de ladite cellule de plante transformée.

2. Procédé selon la revendication 1, **caractérisé en ce que** la séquence nucléotidique code pour l'annexine *NtnaNH12 (SEQ. ID N°1)* ou une portion active de celle-ci.

3. Procédé selon la revendication 1 ou 2, **caractérisé en ce que** la plante est choisie parmi le groupe constitué par les gymnospermes et les angiospermes.

4. Procédé selon la revendication 3, **caractérisé en ce que** la plante est un arbre.

5. Procédé selon l'une quelconque des revendications précédentes, **caractérisé en ce que** la séquence régulatrice est une séquence promotrice et/ou terminatrice active dans une plante.

6. Procédé selon l'une quelconque des revendications précédentes, **caractérisé en ce que** la séquence régulatrice est une séquence régulatrice inducible.

7. Procédé selon l'une quelconque des revendications précédentes, **caractérisé en ce que** la séquence régulatrice comprend une séquence promoteur constitutive ou une séquence promoteur étrangère.

8. Procédé selon l'une quelconque des revendications précédentes, **caractérisé en ce que** la séquence régulatrice est une séquence d'un promoteur choisi parmi le groupe constitué par le promoteur 35S Cauliflower Mosaic Virus ou le promoteur Polyubiquitin Arabidopsis thaliana.

9. Plante transgénique présentant une floraison accélérée, **caractérisée en ce qu'**elle comprend une construction nucléotidique incorporant une séquence nucléotidique codant pour une protéine ou une portion active de ladite protéine de la famille des annexines, ladite séquence nucléotidique étant liée à une ou plusieurs séquences régulatrices actives dans une plante.

10. Plante transgénique selon la revendication 9, **caractérisée en ce que** la séquence nucléoitique code pour l'annexine *NtanNH12 (SEQ. ID N°1)* ou une portion active de celle-ci.

11. Plante transgénique selon la revendication 9 ou 10, **caractérisée en ce que** la séquence régulatrice (de préférence inducible) est une séquence promotrice et/ ou terminatrice active dans une plante.

12. Plante transgénique selon la revendication 11, **caractérisée en ce que** la séquence régulatrice est une séquence d'un promoteur choisi parmi le groupe constitué par le promoteur 35S Cauliflower Mosaic Virus ou le promoteur Polyubiquitin Arabidopsis thaliana.

13. Tissu ou organe de plante transgénique **caractérisé en ce qu'**il est choisi parmi le groupe constitué par les fruits, les bourgeons, les racines, les fleurs ou les semences de la plante tansgénique selon l'une quelconque des revendications précédentes 9 à 12.

14. Structure reproductible obtenue à partir de la plante transgénique selon l'une quelconque des revendications précédentes 9 à 12.


SEQUENCE LISTING

<110> Université Libre de Bruxelles

<120> PLANTE GENETIQUEMENT MODIFIEE ET PRESENTANT UNE FLORAISON ACCELEREE

<130> P.ULB.83/EP

<140> EP 02447267.2
<141> 2002-12-23

<160> 1

<170> PatentIn version 3.1

<210> 1
<211> 954
<212> DNA
<213> Nicotiana tabacum

<400> 1
atggctacaa tcaattaccc tgaaaaccct tctcctgttg ctgatgcaga ggctattagg 60
aaggcttgtc aaggttgggg aacagatgag aaggccataa tttcaatatt tggccataga 120
aatgcaactc aaaagaagct gatcagacga gcttacgagg agttgtacaa tgaagatctt 180
gtgaagcgcc ttgaatctga gttgtctgga cactttgaga aagcagtata cagatggatt 240
ctagatccag aagacagaga tgcagtaata ttgcacgttg caataaaaga gacaccaatt 300
ccagattacc gtgtgattat tgagtactca tgcatctatt cccctgaaga gttgttggct 360
gtaaagcgtg cctatcaagc tcgctacaag cgttctgtgg aggaagattt agctgagcat 420
tctgctggtg aacttcgaaa gcttttggtt gccctagtgg gcatatacag gtatgctggt 480
gaagacataa atgcaagagt agccaacacc gaggccgaca tccttcacag tgcaataagt 540
aacaaggaat tcaataatga agaaattgtt agaatcattt ctacaaggag catccctcaa 600
ctcatagcaa ctctcaaccg ctacaaggat gattatggct cttcaattac caagcacttg 660
agggatgatg caaatgcagc taaggaatac ctagtagcac tgcgtacaac aatccgatgc 720
attagtgacc cccagaaata ctatgaaaag gtaatccggt atgccattaa cgagtctggg 780
actgatgaag agtcagtgac aagagtgata gttacaaggg cagagaagga cttgaaggat 840
atcaaggagc tttactacaa gagaaacagt gtcactcttg atcatgcact ctccaagcac 900

acttctggag attacaaggc tttccttctc gctcttttgg gaaatgataa ctaa          954